# EUROPEAN PATENT SPECIFICATION

(11) **EP 4 042 934 B1**
(45) Date of publication and mention of the grant of the patent: **14.05.2025**
(21) Application number: 20874151.2
(22) Date of filing: 08.10.2020
(51) Int. Cl.: A61B 5/01, A61B 17/00, A61B 90/00

(54) **HEAT-SENSITIVE PLATE FOR QUALITATIVELY RECORDING THE COMPONENT TEMPERATURE OF LIVING ORGANISMS USING HEAT-SENSITIVE SILICONE**
WÄRMEEMPFINDLICHE PLATTE ZUR QUALITATIVEN ERFASSUNG DER KOMPONENTENTEMPERATUR LEBENDER ORGANISMEN UNTER VERWENDUNG VON WÄRMEEMPFINDLICHEM SILIKON
PLAQUE THERMOSENSIBLE À ENREGISTREMENTS QUALITATIFS DE LA TEMPÉRATURE DE COMPOSANT D'ORGANISMES VIVANTS AU MOYEN D'UN SILICIUM THERMOSENSIBLE

(30) Priority: 09.10.2019 MX 2019012160
(43) Date of publication of application: 17.08.2022
(73) Proprietor: De Font-Réaulx-Rojas, Enrique, Ciudad de México, 053000 (MX)
(72) Inventor: De Font-Réaulx-Rojas, Enrique, Ciudad de México, 053000 (MX)
(74) Representative: Spachmann, Holger
(86) International application number: PCT/MX2020/050035
(87) International publication number: WO 2021/071352

(56) References cited:
- WO-A1-2013/177112
- WO-A1-2019/191703
- US-A1- 2008 146 913
- US-A1- 2009 204 100
- US-A1- 2012 157 804
- US-A1- 2018 014 734

## Description

### FIELD OF THE INVENTION

This invention refers to a thermosensitive plate for qualitative measurements of brain temperature in surgeries by means of thermosensitive silicone used in the field of medical surgery.

### BACKGROUND OF THE INVENTION

In current medical treatments for refractory epilepsy that require surgery to control or cure epilepsy, it is essential to be very precise regarding which part of the brain should be removed during surgery to improve control of the crisis, and it is equally important to define which part of the brain it is not necessary to remove during epilepsy surgery.

There are currently methods such as electroencephalogram and magnetic resonance imaging, brain tomography, Single Photon Emission Tomography (SPECT), magnetoencephalogram, and the methods used during epilepsy surgery to define the epileptogenic zones are electrocorticography and stereoelectroencephalography.

Some of the aforementioned methods are invasive and can affect other areas of the patient's brain during surgery. This invention provides a thermosensitive plate that allows qualitative brain temperature measurements to be taken during surgery using a thermosensitive siliconethat changes color according to the temperature to which it is exposed, which will allow the neurosurgeon to visually identify different cerebral cortical temperature patterns.

This thermosensitive plate may also be used in other areas of medicine, both surgical and non-surgical, in which it is wished to observe the different temperature patterns of the surfaces with which it is in contact, and in non-medical areas.

The same device described above also has incorporated electrodes to simultaneously perform a bimodal record that includes the electrocorticography and the qualitative thermography by means of the change in color of the thermosensitive silicone.

The same device described above also has materials incorporated to be registered and followed by navigation systems used in surgery.

WO 2019/191703 A1 discloses a wireless skin sensor configured to remotely observe a patient. The sensor comprises a silicone plate which is connected with several sensory electronics to form the sensor. The sensor is configured to be applied on the skin of the patient.

US 2018/0014734 A1 discloses a device for analysis of temperature and thermal transport characteristics of the skin. The device includes a flexible substrate with several sensors and thermal actuators attached. The thermal actuators stimulate the skin, while induced temperature changes can be visualized through pixels.

Wo 2013/177112 A1 discloses a thermochromic patch for monitoring the body temperature. The patch may be fabricated of fiber or similar material and may comprise a thermochromic agent to visualize the temperature through color.

US 2009/0204100 A1 discloses a body cover applicable on the skin. The cover may be formed by a flexible substrate and comprises a sensing element, which can be formed by a thermochromic material. The cover is configured to visualize the skin temperature, for example to identify inflammation regions (paragraphs [0016-0018], claims 2, 15, 18).

### DETAILED DESCRIPTION OF THE INVENTION

### BRIEF DESCRIPTION OF THE FIGURES

- **Figure 1**: illustrates the parts of the thermosensitive plate, showing the basic version of the thermosensitive plate.
- **Figure 2**: illustrates a thermosensitive/thermochromic plate with incorporated electrodes to obtain bimodal information (qualitative temperature and electrical activity of the organ or tissue).
- **Figure 3**: illustrates a translucent or thermosensitive/thermochromic plate that is compatible with navigation systems.
- **Figure 4**: is a photo of case 1.

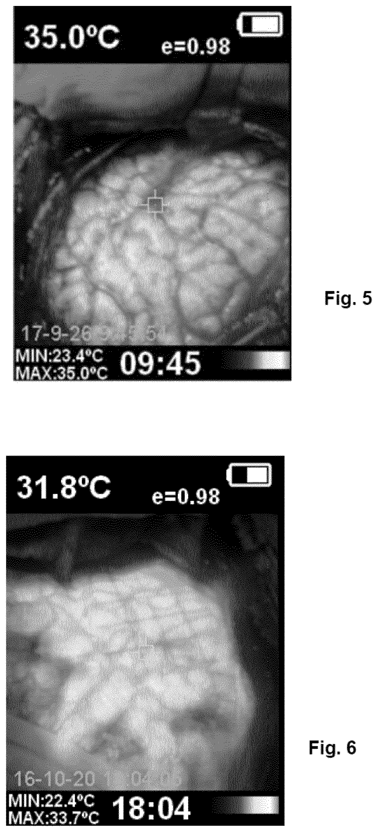

- **Figure 5**: is a photo of case 2.
- **Figure 6**: is a photo of case 3.

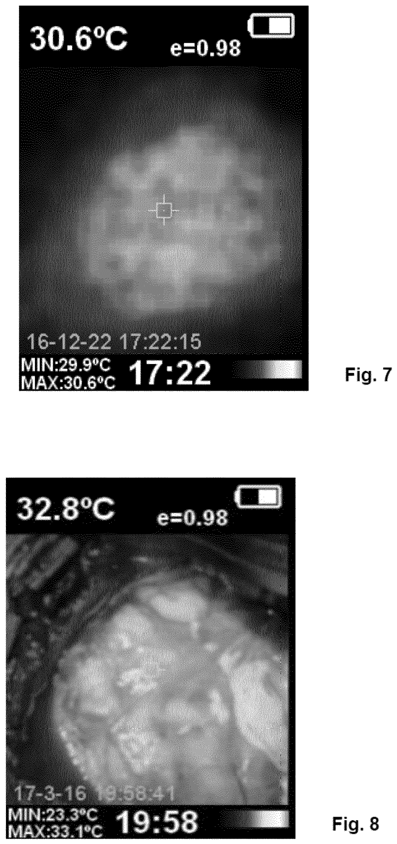

- **Figure 7**: is a photo of case 4.
- **Figure 8**: is a photo of case 5.

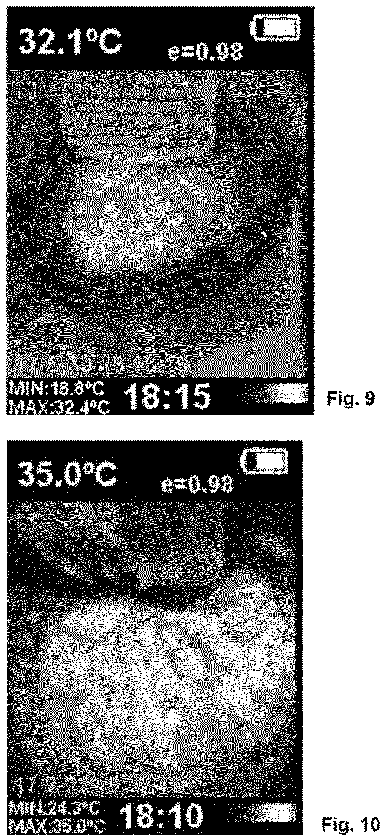

- **Figure 9**: is a photo of case 6.
- **Figure 10**: is a photo of case 7.

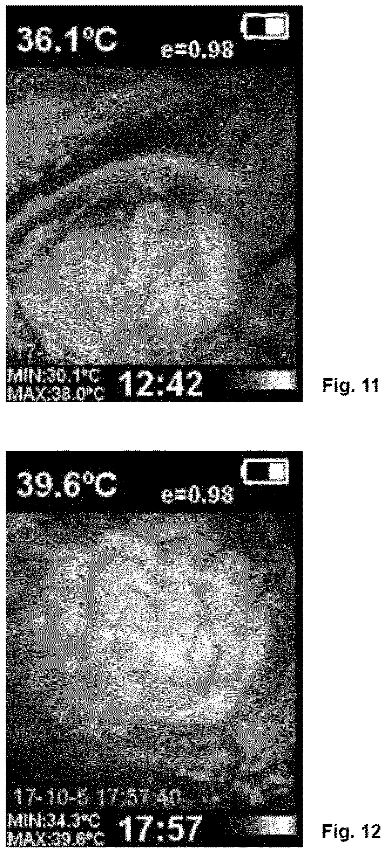

- **Figure 11**: is a photo of case 8.
- **Figure 12**: is a photo of case 9.

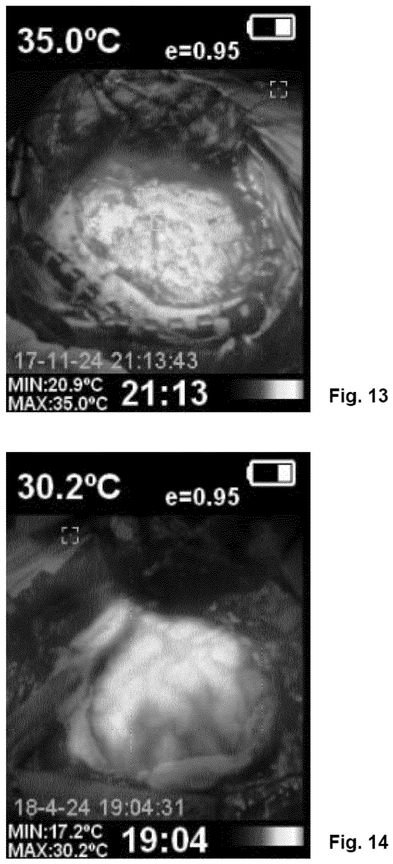

- **Figure 13**: is a photo of case 10.
- **Figure 14**: is a photo of case 11.
- **Figure 15**: is a photo of case 12.
- **Figure 16**: is a photo of case 13.
- **Figure 17**: illustrates a dispersion graph between "Irritative Zone Temperature" and "Maximal Brain Temperature."
- **Figure 18**: illustrates a trivariate histogram between Age, Age of Onset, and Meningeal Temperature.
- **Figure 19**: illustrates a trivariate analysis graph between Age, Star Age, and Delta T.
- **Figure 20**: illustrates a graph showing the difference of medians between Registered Temperatures.
- **Figure 21**: illustrates a graph that shows the difference of medians between the Age Proportion/Time with Epilepsy.

### DETAILED DESCRIPTION OF THE INVENTION

The present invention is disclosed in the appended claims.

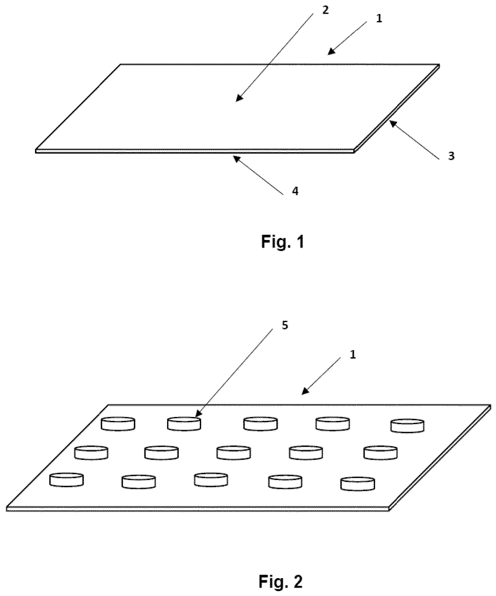

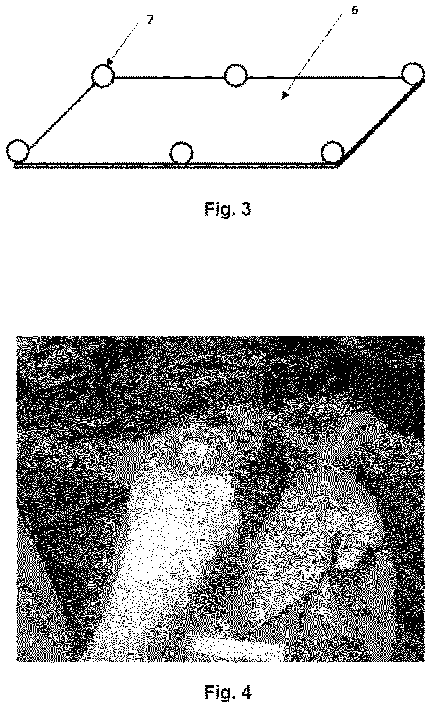

We have discovered that the epileptogenic zones are colder than the rest of the brain, as their metabolism is different. There is currently not a thermosensitive plate specifically designed to identify the thermogenic metabolic behavior of the brain during neurosurgical procedures. The thermosensitive plate of this invention could be used in different neurosurgical procedures, such as: epilepsy surgery, vascular neurosurgery, and oncological neurosurgery, among others, and in general surgery to monitor the temperature of organs during transplants, during cardiac bypass surgery, oncological surgery, etc.; furthermore, it may also be used in fields outside of medicine. As shown in Figures 1-3, this invention comprises a thermosensitive silicone plate (1) that responds to the different temperatures to which it is exposed, exhibiting color changes within a few seconds. The basic plate may be phosphorescent, as well as thermosensitive and phosphorescent.

The thermosensitive plate is be made of flexible and non-adhesive polymers, one of which is be silicone. This raw material is combined with a colorant, commonly in powder form, but it may be made of any presentation that can be mixed with the base that will form the plate, and once mixed, a catalyst is added and it is poured into a mold. The speed of plate response and the color change contrast once it has come into contact with the thermogenic surface, depends on the quantity of the colorant and the thickness of the plate.

More specifically, the chemical components of the thermosensitive plate are "RTV" (room temperature vulcanized) type liquid silicone, to which 0.1 to 30% (range not limited) of the powdered thermochromic thermosensitive colorant is added, which responds with thermal changes within the range of the temperature of the live organism on which it will be placed, and once these two initial components have been mixed homogenously, 3% of the catalyst is added, which is calculated in relation to the quantity of silicone used. It is then mixed homogenously and poured into the mold, which may be in any shape, but preferably flat so that the thermosensitive/thermochromic silicone has the largest surface of contact with the target organ or tissue. The thinner the transfer/thickness surface, the more quickly the thermosensitive/thermochromic silicone will respond and with better spatial resolution. In the claimed embodiment of the invention, the thickness range is 1 to 5 mm. Once the drying process at ambient temperature has finished over a maximum period of 48 hours, it may be removed from the mold and cut to the desired size to cover the organ or tissue with which it will be in contact.

The thermosensitive plate changes color within a range that can be easily seen by the naked eye, without requiring any type of special light or filter. Normally colors are chosen that contrast when they change color: white/black, purple/pink, yellow/green. The speed of response depends in large part on the amount of colorant that is incorporated into the base plate, and the thickness of the plate. In the claimed embodiment of the inventionwhen the thermosensitive plate comes into contact with the target surface, organ, or tissue, the thermosensitive/thermochromic silicone will change color in a few seconds (depending on the thickness and the quality of the colorant that is included in the mixture; the more colorant there is (without saturating the mixture) and the thinner the plate, the response will have better spatial distribution and will occur in a shorter period of time). Preferably two colors will be chosen (although it is not limited to being two-color) that easily show contrast to the naked eye (including, but not limited to: purple to Mexican pink) as shown in the images. In living beings, higher metabolism usually generates higher temperatures, and vice versa, thus the thermosensitive/thermochromic silicone plate according to the invention may have several patterns, depending on the temperature to which it is exposed.

The thermosensitive/thermochromic plate (1) may be applied directly to the surface of the target tissue or organ to create functional maps, such as over a cerebral cut to identify the zones of metabolic activation of eloquent areas or areas with different metabolism, such as, for example, epileptogenic zones and the start of ictal zones due to their different metabolism. It may also be used to identify neoplastic or infectious lesions on any organ, without the need to use contrast dyes or media. This same thermosensitive/thermochromic plate (1) may have localizers for the navigation systems and/or electrodes for bimodal registration.

The thermosensitive silicone plate (1) may have incorporated electrodes (5) to simultaneously perform electrocorticography, and for qualitative determination of the cerebral temperature.

More specifically, the thermosensitive plate for qualitative measurements of the component temperature of organisms of this invention comprises a thermosensitive silicone plate (1) with a temperature irradiation surface (2), a conduction plane (3), and a temperature transfer surface (4); and at least one electrode to register electrical activity (5) placed on the temperature irradiation surface (2).

The device of this invention may also comprise compatible material locators (7) to be detected by navigation systems placed on the temperature irradiation surface (2), with the goal of the navigation system being able to make corrections in rotation, movement, and deformation of the target tissue or organ.

With the technical characteristics of this invention, the observer will be able to visually identify different temperature patterns with which the device is in contact. The silicone may also be transparent, with color, or it may be thermochromic, and it may also have "fiducials" incorporated into it, which are markers that can be recognized by the different navigation systems that will allow the navigation systems to make adjustments through anatomic changes caused by surgical manipulation of the tissue or organ that is manipulated, or by displacement by gravity, movement, rotation, or anesthetic effect, among others, thus increasing precision during navigation-guided surgeries. The thermosensitive/thermochromic silicone may also be phosphorescent.

With the thermosensitive/thermochromic silicone plate of the thermosensitive plate of this invention, functional maps can be made of the different components and organs of living beings, including functional cerebral mapping.

The thermosensitive/thermochromic silicone plate of the thermosensitive plate may be flexible and moldable, and it may be placed directly on the surface that will be evaluated, or it may be isolated by a thin layer of material to make it biocompatible, if so required. Furthermore, with the thermosensitive plate of this invention, the blood flow can be detected by its temperature gradient. The thermosensitive plate of this invention does not require electric parts, in its version to be used as the base for electrocorticography grids. The difference with the current electrocorticography grids is that in addition to the electrodes to register the electrical activity of the cerebral cortex, it may have "fiducials" to be detected by navigation systems, and it may be thermosensitive/thermochromic to take BIMODAL measurements of the activity of the organ being evaluated, specifically the nervous system, which includes the qualitative evaluation of the temperature and the electrical activity, SIMULTANEOUSLY.

This invention has a method of application that consists of the steps of:
a) Keeping the thermosensitive plate cold.
b) Eliminating any substance that might interfere in the plate's contact with the target surface.
c) Leaving the plate in contact with the target surface, without moving it or modifying its temperature with solutions or exposure to warm light, for example.
d) Observing the visual response with the naked eye, which will depend on the temperature and adequate contact of the thermosensitive plate with the target surface.
e) Removing the thermosensitive plate.
f) Cooling the plate to a temperature that is lower than body temperature, ideally using a cold sterile saline solution.
g) Repeating the process as required.

An example of an embodiment of the invention that shows that the invention can be used is found in the following epilepsy surgery tests to define the epileptogenic zones, which consists of temperature measurement tables that were created in twelve epilepsy cases, where it can be clearly seen that the zones responsible for the epilepsy are between 2 and 7°C colder than the rest of the brain. That is to say, the temperature varies according to the region. The cortex is colder than the basal ganglia, and the basal ganglia are colder than the brain stem in resting state, and when an eloquent area of the brain is used, this increases temperature, as shown in the videos of the patient on whom I operated when he was awake.

These tables show the measurements from each case, with each table showing a range of colors that clearly illustrates how the irritative activity zone in patients with epilepsy is colder (blue), and around this colder zone there is a radial heating pattern towards the healthy brain tissue. It is from that assessment that a silicone was designed, so that instead of all of these measurements we can qualitatively identify the coldest zone with the change in color of the silicone (purple is cold, pink is hot).

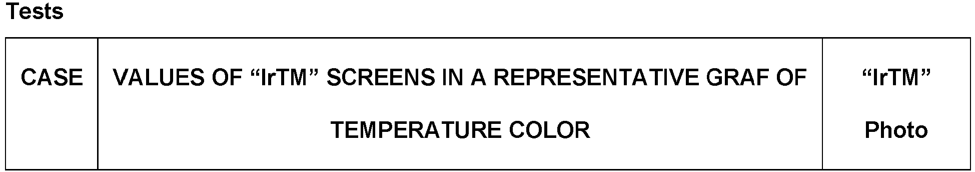

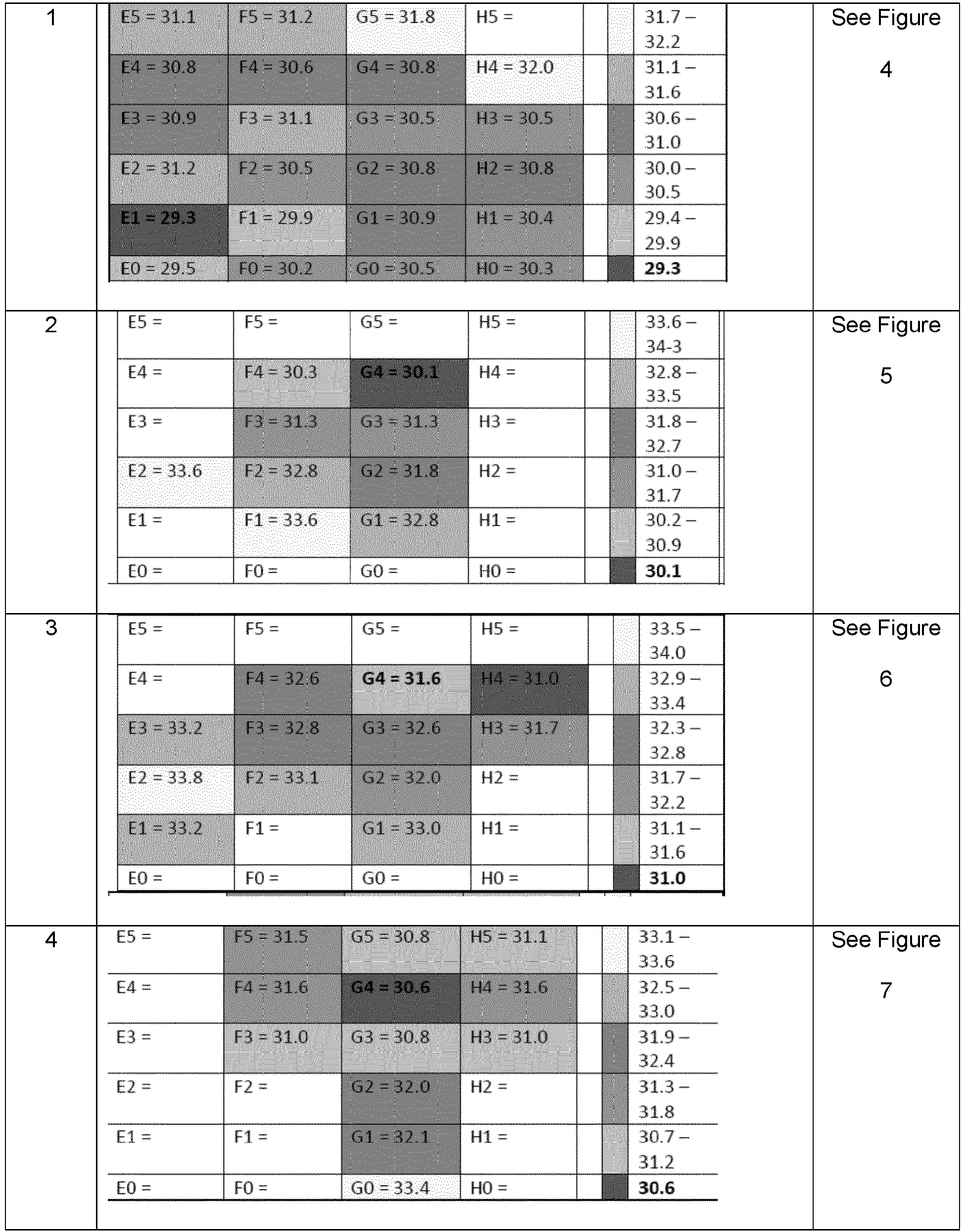

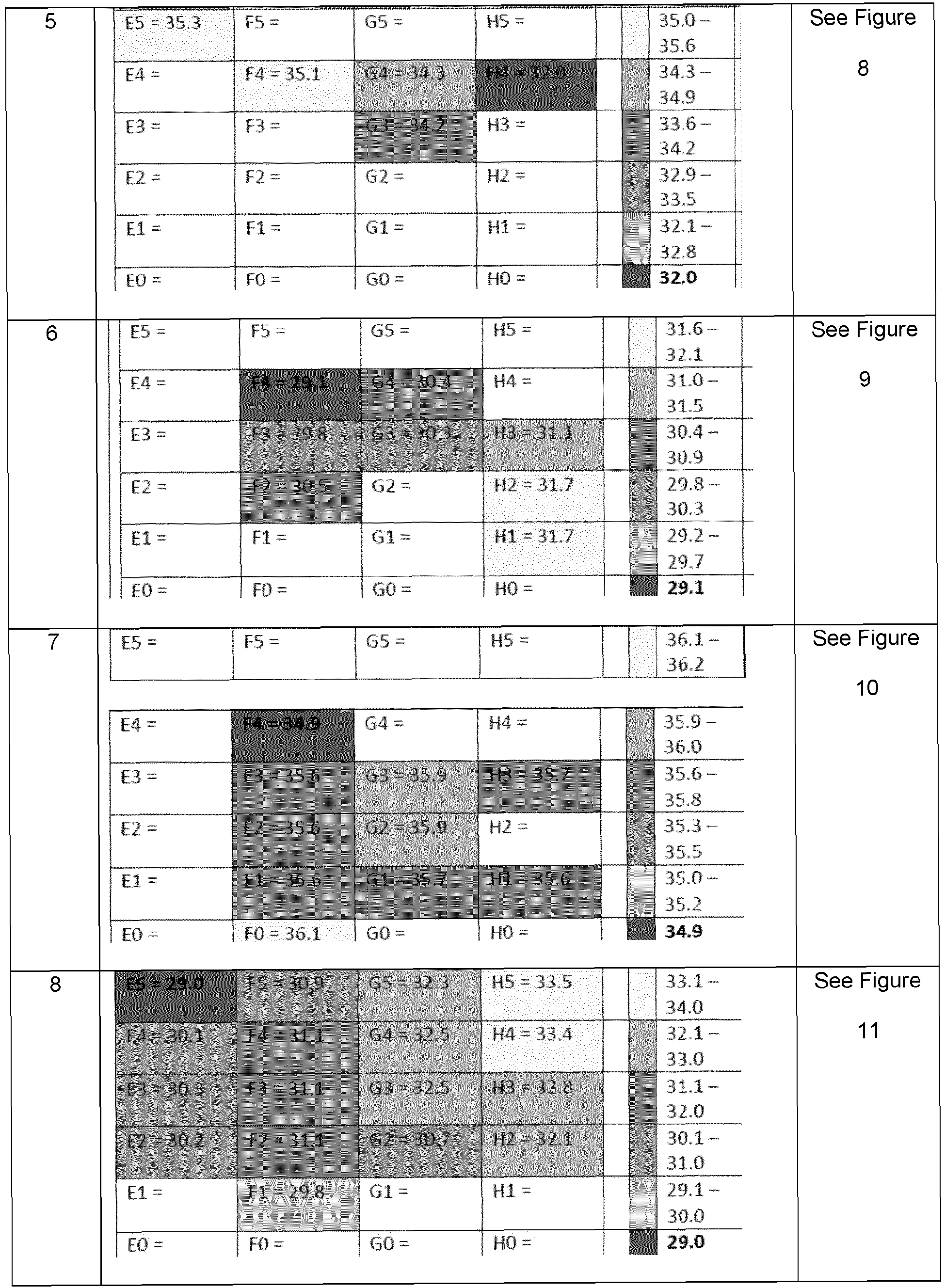

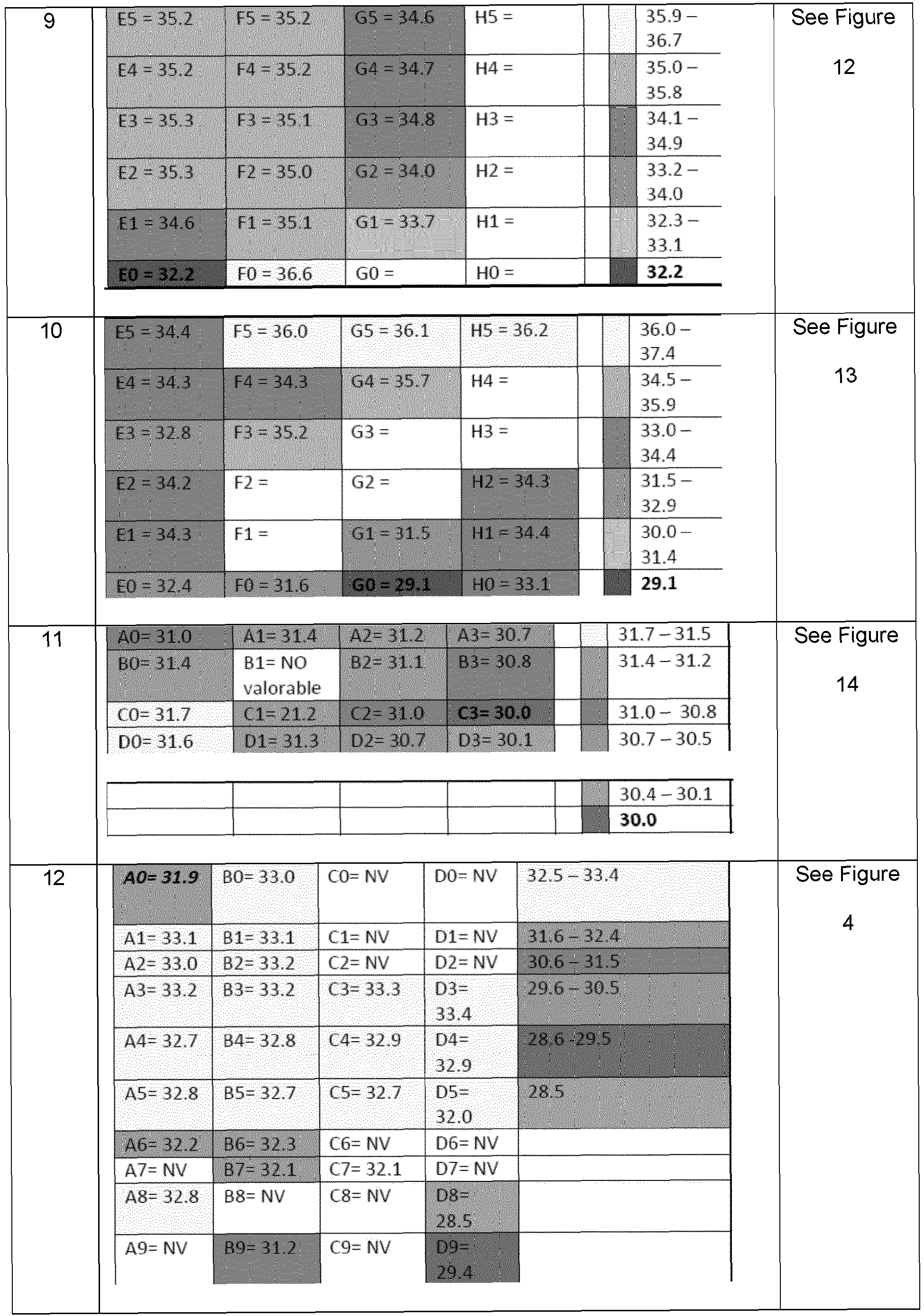

Twelve patients who met the study's inclusion criteria were studied, and of these patients, three were male (25%) and nine were female (75%). The average age was 32.5 ± 16.6596 years, the asymmetry index value was 0.795, therefore the distribution is skewed to the right, with a Kurtosis value of 0.047 being the flat distribution due to the range and size of the population. Note that the average and standard deviation of the temperatures recorded in the Irritative Zone Temperature and the Maximal Brain Temperature are almost equal, therefore the size of the existing sample is valid for this study (see Table 1).

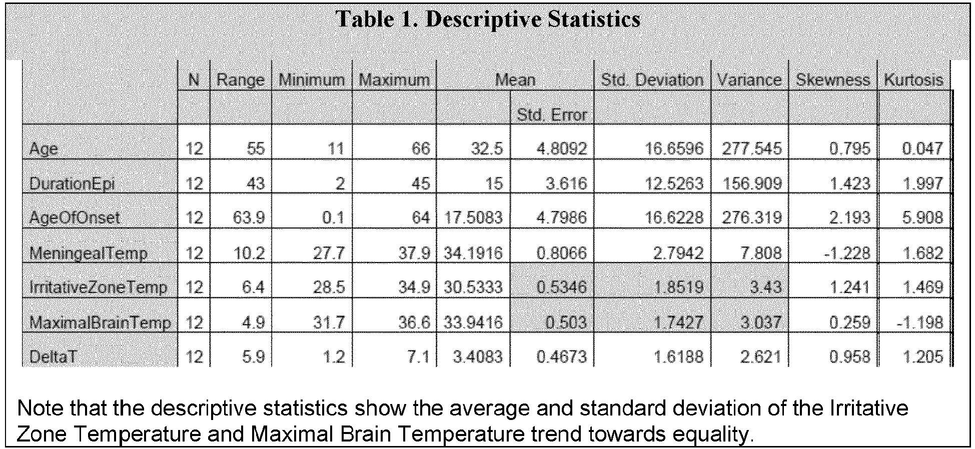

The averages of the recorded temperatures were compared, and a significant difference was found between the Irritative Zone Temperature and the Maximal Brain Temperature with α≤0.0001, and between the Meningeal Temperature and Irritative Zone Temperature with α≤0.004 (see Graph 1). When creating the dispersion graph, a correlation was found (being greater in the interval 300≤x≤400) between the variables used, therefore the Pearson correlation coefficient between these two variables was calculated finding that r=0.596, thus by increasing the temperature in the Irritative Zone Temperature, it is also increased in the Maximal Brain Temperature (see Graph 2). Note that in addition to the correlation that exists between these two variables, the difference between their medians is highly significant.

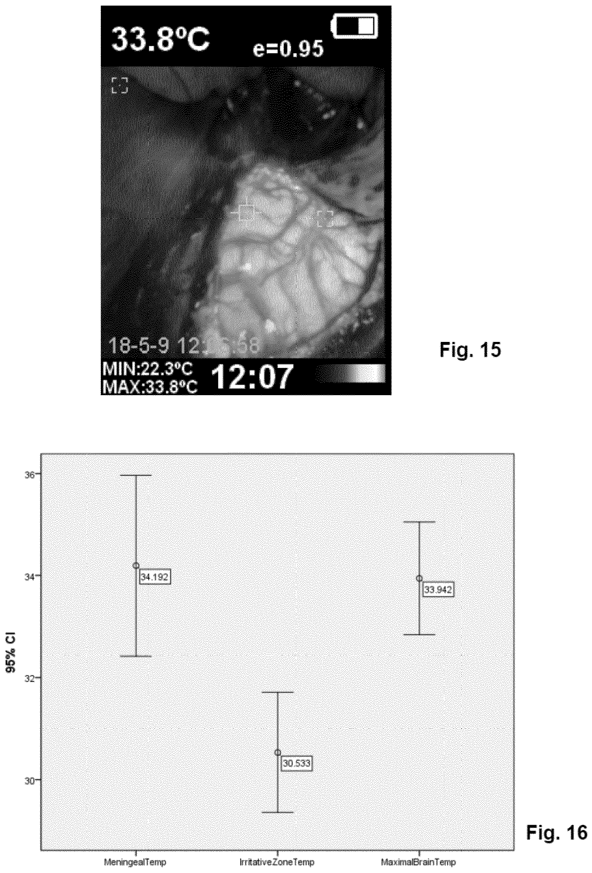

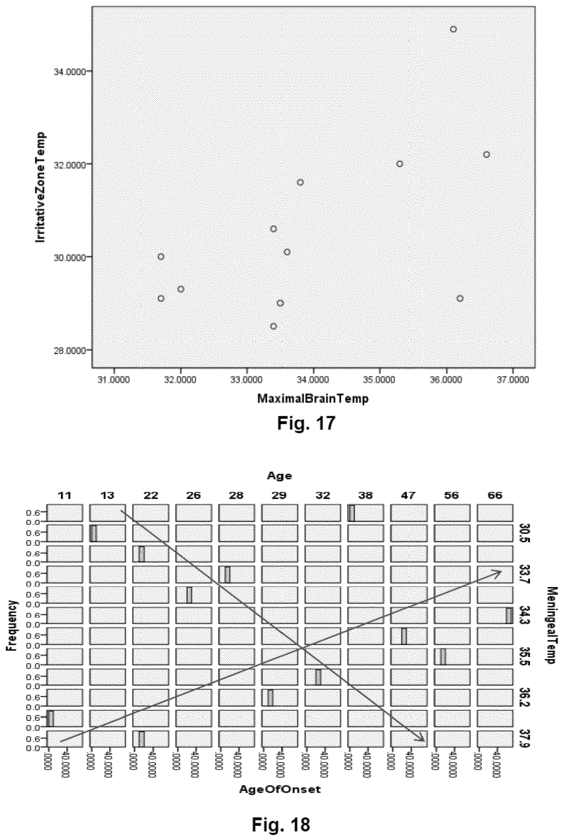

In Figure 16, referring to the graphs of recorded Temperature Errors, it was found that the averages and dispersions where the values with the lowest recorded temperatures were in the Irritative Zone. In Figure 17, referring to the dispersion between the Irritative Zone Temperature and the Maximal Brain Temperature, it is seen that when the temperature in the Irritative Zone is increased, it increases the Maximal Brain Temperature.

A trivariate graph was created in order to analyze the behavior between Age, Meningeal Temperature, and Age of Onset, where two trends are seen based on this analysis: when age increases, the Meningeal Temperature increases in the range of 13-28 years, and likewise it is seen that in the range of 22-56 years, when age increases the Meningeal Temperature decreases (see Graph 3).

In Figure 18, referring to the trivariate histogram between Age, Age of Onset, and Meningeal Temperature, it is seen that temperature tends to stabilize at higher ages.

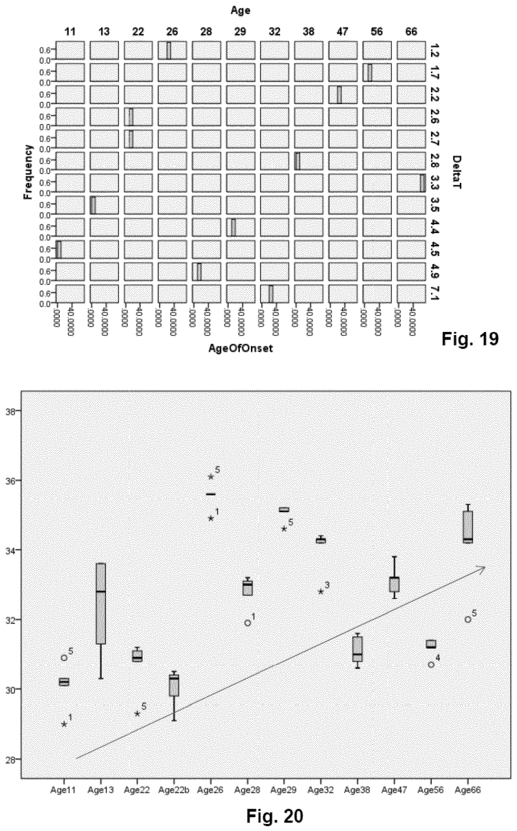

As can be seen in Figure 19, a trivariate graph was created using Age, Age of Onset, and Delta **T,** in order to evaluate the behavior between these three variables. It was found that by increasing the age of the patients, the value of Delta decreases, noting that the Age of Onset does not influence this trend, and when the age of patients increases the value of Delta decreases.

In order to evaluate the behavior of all temperatures recorded by age and patient, a box plot was created in order to compare the medians based on the second and third quartiles, where it is seen that in the interval (26≤x≤56), the value of the medians decreases with respect to age, as shown in Figure 20, where it is seen that as of 26 years of age, the medians of temperature registered decreases.

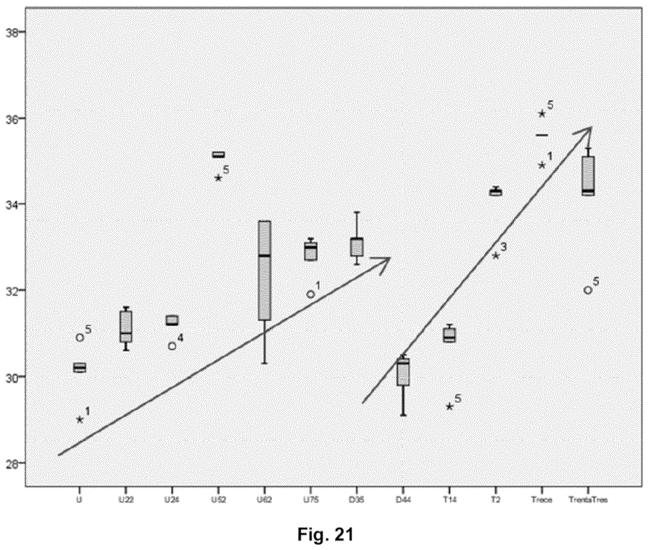

In order to evaluate a possible association between age and time with epilepsy, the proportion was calculated (age/time of epilepsy) and box plots were created with all temperatures recorded to analyze the behavior of the medians based on the second and third quartiles, where it was found that of the proportion in the interval (1(U)≤x≤2.35(D35)) the trend is growing with an angle tending towards 45°, otherwise in the interval (2.44(D44)≤x≤33) the trend is growing with an angle of inclination tending towards 90°, therefore there is a relationship and trend in this proportion, as shown in Figure 21, where the difference can be seen in the trends in the medians of the proportions (U-D35) and (D44-33).

Another test was run during another surgery, in which a bimodal measurement was taken in which electrocorticography and cerebral temperature were registered simultaneously, finding the points of maximum abnormal electrical activity by electrocorticography, which were electrodes 7 and 11, and subsequently the purple-colored thermosensitive plate was placed to determine if in positions 7 and 11 the heat is dissipated and the purple-colored thermosensitive plate turns the color pink in the zone of normal electrical activity, and it remains purple in the zone of equal abnormal electrical activity. That is, it was seen that when the thermosensitive silicone plate (1) was placed in the zone responsible for the epilepsy it remained cold (purple). In that patient it corresponds with the abnormal electrical activity registered with electrodes (electrocorticography) during the surgery, while the rest of the silicone heated up quickly and took on a brilliant pink color in the zone where the electrical activity is normal.

## Claims

1. A thermosensitive plate for qualitative measurements of brain temperature in surgeries, which includes:
a thermosensitive silicone plate (1) with a temperature irradiation surface (2), a conduction plane (3) and a temperature transfer surface (4),
wherein the thermosensitive plate is comprised of: liquid silicone; thermosensitive/thermochromic colorant in powder form of 0.1 to 30%; a catalyst in a quantity of 3%, which is calculated in relation to the quantity of silicone used,
wherein the range of plate thickness is from 1 to 5 mm,
so that the thermosensitive plate is configured in such way, that it shows several visually identifiable temperature patterns defining functional maps when it is in contact with said brain, depending on the temperature to which it is exposed, and does not require any electrical parts.

2. The thermosensitive plate for qualitative measurements of brain temperature in surgeries according to claim 1, wherein it comprises locators of compatible material (7) to be detected by navigation systems placed on the temperature irradiation surface (2).

3. The thermosensitive plate for qualitative measurements of brain temperature in surgeries according to claim 1, wherein it takes qualitative measurements of the cerebral temperature during surgery.

4. The thermosensitive plate for qualitative measurements of brain temperature in surgeries according to claim 1, wherein it detects the blood flow by its temperature gradient.

5. The thermosensitive plate for qualitative measurements of brain temperature in surgeries according to claim 1, wherein it has at least one electrode to record electrical activity (5) placed on the temperature irradiation surface (2).

6. A method for preparing a thermosensitive plate for qualitative measurements of brain temperature in surgeries according to, which comprises the steps of:
Adding liquid silicone in a recipient, to which 0.1 to 30% of the thermosensitive/thermochromic colorant in powder form is added, which responds with thermal changes within the temperature range of a brain on which it will be placed, and once the homogenous mixture of these two initial components has been done, 3% of a catalyst is added, which is calculated in relation to the amount of silicone used; then it is homogenously mixed and poured into a mold so that a range of a resulting plate thickness is from 1 to 5 mm, which may be in any shape, but preferably flat so that the thermosensitive/thermochromic silicone has the largest surface for contact with the target brain; and once the drying process at ambient temperature has finished within a maximum period of 48 hours, it may be removed from the mold and cut to the size desired to cover the brain with which it will be in contact

## Patentansprüche

1. Eine wärmeempfindliche Platte zur qualitativen Messung der Gehirntemperatur bei Operationen, die Folgendes umfasst:
eine thermosensitive Silikonplatte (1) mit einer Temperaturbestrahlungsfläche (2), einer Leitungsfläche (3) und einer Temperaturübertragungsfläche (4),
wobei die thermosensitive Platte folgendes umfasst: flüssiges Silikon;
thermosensitiver/thermochromer Farbstoff in Pulverform von 0,1 bis 30 %; einem Katalysator in einer Menge von 3 %, berechnet im Verhältnis zur verwendeten Silikonmenge, wobei der Bereich der Plattendicke zwischen 1 und 5 mm liegt,
sodass die thermosensitive Platte so konfiguriert ist, dass sie bei Kontakt mit dem Gehirn je nach der Temperatur, der sie ausgesetzt ist, mehrere visuell erkennbare Temperaturmuster anzeigt, die funktionelle Karten definieren, und dass sie keine elektrischen Teile benötigt.

2. Die thermosensitive Platte für qualitative Messungen der Gehirntemperatur bei chirurgischen Eingriffen gemäß Patentanspruch 1, wobei sie Positionierungshilfen aus kompatiblem Material (7) umfasst, die von Navigationssystemen erkannt werden können, die auf der Temperaturbestrahlungsoberfläche (2) platziert sind.

3. Die thermosensitive Platte zur qualitativen Messung der Gehirntemperatur bei Operationen gemäß Patentanspruch 1, wobei sie während der Operation qualitative Messungen der Gehirntemperatur vornimmt.

4. Die thermosensitive Platte zur qualitativen Messung der Gehirntemperatur bei Operationen gemäß Patentanspruch 1, wobei sie den Blutfluss anhand ihres Temperaturgradienten erkennt.

5. Die thermosensitive Platte zur qualitativen Messung der Gehirntemperatur bei Operationen gemäß Patentanspruch 1, wobei sie mindestens eine Elektrode zur Aufzeichnung elektrischer Aktivität (5) aufweist, die auf der Temperaturbestrahlungsfläche (2) platziert ist.

6. Ein Verfahren zur Herstellung einer thermosensitiven Platte für qualitative Messungen der Gehirntemperatur bei chirurgischen Eingriffen, das folgende Schritte umfasst:
Zugabe von flüssigem Silikon in einen Behälter, dem 0,1 bis 30 % des thermosensitiven/thermochromen Farbstoffs in Pulverform zugesetzt werden, der auf thermische Veränderungen innerhalb des Temperaturbereichs eines Gehirns reagiert, auf das dieses gelegt wird, und sobald die homogene Mischung dieser beiden Ausgangskomponenten erfolgt ist, werden 3 % eines Katalysators zugesetzt, der im Verhältnis zur verwendeten Silikonmenge berechnet wird; dann wird es homogen gemischt und in eine Form gegossen, so dass eine resultierende Plattendicke von 1 bis 5 mm reicht, die jede beliebige Form haben kann, aber vorzugsweise flach ist, damit das thermosensitive/thermochrome Silikon die größte Kontaktfläche mit dem Zielhirn hat; und sobald der Trocknungsprozess bei Umgebungstemperatur innerhalb einer Zeitspanne von höchstens 48 Stunden abgeschlossen ist, kann es aus der Form entfernt und auf die gewünschte Größe zugeschnitten werden, um das Gehirn abzudecken, mit dem es in Kontakt kommen wird

## Revendications

1. Une plaque thermosensible pour les mesures qualitatives de la température cérébrale lors d'interventions chirurgicales, qui comprend :
une plaque de silicone thermosensible (1) avec une surface d'irradiation de température (2), un plan de conduction (3) et une surface de transfert de température (4),
dans laquelle la plaque thermosensible est composée de : silicone liquide ; colorant thermosensible/thermochromique sous forme de poudre de 0,1 à 30 % ; un catalyseur en une quantité de 3 %, qui est calculée par rapport à la quantité de silicone utilisée, dans laquelle l'épaisseur de la plaque est comprise entre 1 et 5 mm,
de sorte que la plaque thermosensible est configurée de telle sorte qu'elle présente plusieurs modèles de température visuellement identifiables définissant des cartes fonctionnelles lorsqu'elle est en contact avec ledit cerveau, en fonction de la température à laquelle elle est exposée, et ne nécessite aucune pièce électrique.

2. Plaque thermosensible pour la mesure qualitative de la température cérébrale en chirurgie selon la revendication 1, dans laquelle elle comprend des localisateurs de matériau compatible (7) à détecter par des systèmes de navigation placés sur la surface d'irradiation de température (2).

3. Plaque thermosensible pour mesures qualitatives de la température cérébrale lors d'interventions chirurgicales selon la revendication 1, dans laquelle elle prend des mesures qualitatives de la température cérébrale pendant l'intervention chirurgicale.

4. Plaque thermosensible pour mesures qualitatives de la température cérébrale en chirurgie selon la revendication 1, dans laquelle elle détecte le flux sanguin par son gradient de température.

5. Plaque thermosensible pour mesures qualitatives de la température cérébrale lors d'interventions chirurgicales selon la revendication 1, **caractérisée en ce qu'**elle comporte au moins une électrode pour enregistrer l'activité électrique (5) placée sur la surface d'irradiation de température (2).

6. Procédé de préparation d'une plaque thermosensible pour des mesures qualitatives de la température cérébrale lors d'interventions chirurgicales, qui comprend les étapes suivantes :
Ajouter du silicone liquide dans un récipient, auquel on ajoute 0,1 à 30 % du colorant thermosensible/thermochromique sous forme de poudre, qui réagit aux variations thermiques dans la plage de température d'un cerveau sur lequel il sera placé, et une fois le mélange homogène de ces deux composants initiaux effectué, on ajoute 3 % d'un catalyseur, qui est calculé en fonction de la quantité de silicone utilisée ; ensuite, on mélange de manière homogène et on verse dans un moule de manière à obtenir une épaisseur de plaque résultante comprise entre 1 et 5 mm, qui peut être de n'importe quelle forme, mais de préférence plate, de sorte que le silicone thermosensible/thermochromique ait la plus grande surface de contact avec le cerveau cible ; et une fois le processus de séchage à température ambiante terminé dans un délai maximum de 48 heures, on peut le démouler et le découper à la taille souhaitée pour recouvrir le cerveau avec lequel il sera en contact
